# EUROPEAN PATENT APPLICATION

(11) **EP 1 136 045 A2**
(43) Date of publication of application: **26.09.2001**
(21) Application number: 01302095.3
(22) Date of filing: 07.03.2001
(51) Int. Cl.: A61F 2/30, A61F 2/38

(54) **Prosthesis with securing flange**

(30) Priority: 21.03.2000 GB 0006844
(71) Applicant: Biomet Merck Limited, Bridgend, South Glamorgan CF31 3XA (GB)
(72) Inventor: Bateman, Ronald, Nr. Purton, Swindon SN5 9LA (GB); Murray, David, Horspath, Oxford OX33 1RU (GB); O'Connor, John, Headington, Oxford OX3 8JN (GB); Goodfellow, John, Summertown, Oxford OX2 7RU (GB)
(74) Representative: Giles, Ashley Simon

(57) **Abstract**

A prosthesis (1) for attachment to a bone, the prosthesis (1) comprising: a body portion (2) having a substantially flat bone engaging region (4); a substantially straight flange (12); and a web (14) which connects the flange (12) to the bone engaging region (4) of the body portion (2) and spaces it away from the bone engaging region (4) in such a way that the flange (12) is not parallel to the bone engaging region (4).

## Description

### BACKGROUND TO THE INVENTION

In osteoarthritis, the disease process usually starts in one of the tibio-femoral compartments and only spreads to involve the other compartment at a late stage in its development. Experience has shown that total replacement of one compartment is all that is needed to provide prolonged relief from symptoms.

In a conventional procedure to attach a uni-compartmental tibial implant to the proximal end of the human tibia, the tibial plateau is excised using two saw cuts and a tibial groove prepared. Cement is pressed into the tibial groove and spread in a thin layer on the flat surface of the tibia. The tibial implant is then placed into position and tapped home using a suitable punch. Excess cement is carefully removed from all around the tibial implant, particularly posteriorly.

It has been observed that the use of cement in such a uni-knee arthroplasty may have disadvantages.
More. specifically, there may be a higher risk of infection with cemented implants, and also cement particles can work loose, and result in third body wear. The infection risk is increased partly because of the longevity of the surgery. The present invention has been developed to provide an improved method of securing the implant in the tibia which minimises, or avoids altogether, the use of cement.

### SUMMARY OF THE INVENTION

According to a first aspect of the present invention there is provided a prosthesis comprising a body portion having a substantially flat bone engaging region, a flange, and a web which connects the flange to the bone engaging region of the body portion and spaces it away from the bone engaging region in such a way that the flange is not parallel to the bone engaging region.

Preferably, the flange is substantially straight.

Preferably, the bone engaging region comprises a plurality of bone engaging surfaces disposed substantially in a common plane.

Preferably, the widest part of the web is narrower than the widest part of the flange when viewed in a plane perpendicular to a longitudinal axis of the flange. The web may be discontinuous. For example, one or more openings may be provided through the web, the opening(s) permitting bone growth through the web to improve the anchorage of the prosthesis in the bone.

Preferably, the flange is of circular cross section. Most preferably, the flange has a cross-section defined by a segment of a circle adjoining and coincident with a segment of a circle of greater diameter. In this way, the implant can be effectively prestressed into firm contact with the tibia without the need for cement.

Preferably, the bone engaging region is coated in porous hydroxyapatite material, to encourage bone growth and hence formation of a strong bond between the implant and bone.

### BRIEF DESCRIPTION OF THE DRAWINGS

For a better understanding of the present invention and to show how it may be carried into effect reference will now be made, by way of example, to the accompanying drawings, in which:-
Figure 1 is a side view, partially in cross-section, of a knee prosthesis having a keel constructed in accordance with the present invention;
Figure 2 is a view from above, partially in cross-section, of the knee prosthesis shown in Figure 1; and
Figure 3 is an end view, partially in cross-section, of the knee prosthesis shown in Figure 1.

Figure 4 is a side view, partially in cross-section, of a second embodiment of knee prosthesis.

### DETAILED DESCRIPTION OF THE PREFERRED EMBODIMENTS OF THE INVENTION

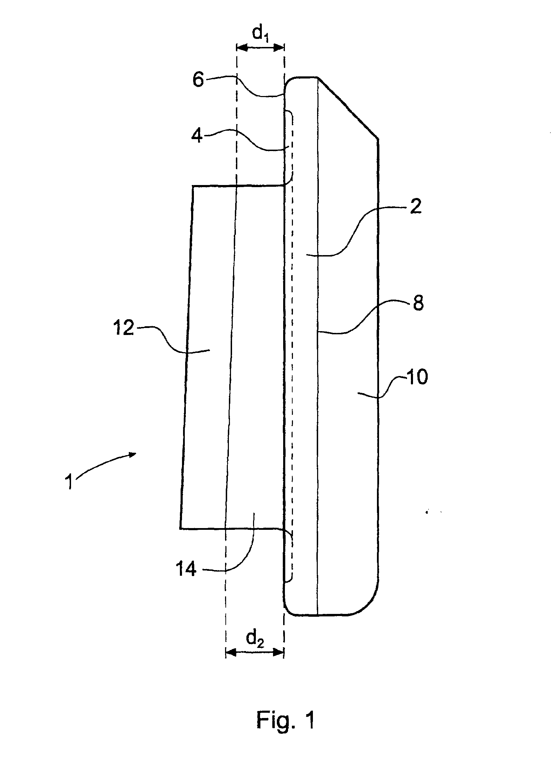

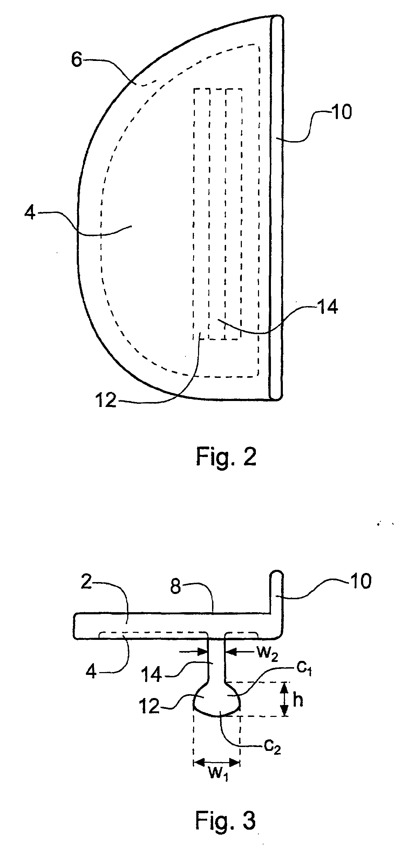

Figures 1 and 2 show a prosthesis 1 used to replace a single condylar bearing portion of a human tibia.

The prosthesis 1 comprises a body portion 2 having a substantially flat bone engaging region 4 which has a raised border disposed around its periphery defining a bone engaging surface 6. The body portion 2 provides an articular surface 8 having an abutment 10 projecting therefrom to form a tibial tray. In use the articular surface 8 supports a meniscal component (not shown) for engagement with a prosthetic femoral component (not shown) in such a manner as to enable the prosthesis to serve as a replacement for the natural tibial articular surface and to restore substantially normal anatomical movement of the femur and tibia. As is well known in the art the abutment 10 prevents dislocation of the meniscal component towards the cruciate ligaments.

The bone engaging region 4 is preferably coated in hydroxyapatite or similar porous material for encouraging bone growth, whilst the body portion 2 is constructed of a metal compatible with use in the body. The preferred material for the meniscal component is high-density polyethylene, which provides the articular surface with the appropriate lubricity characteristics for the proper functioning of the knee prosthesis.

The body portion 2 is fixed to the tibia by means of an elongate flange 12 which is anchored directly in the tibia. The flange 12 is connected to the bone engaging region 4 of the body portion 2 by means of a continuous web 14 which is narrower than the flange 12 and is tapered in a longitudinal direction of the flange 12. Because the web 14 is tapered it spaces the flange 12 away from the bone engaging surface 6 by a distance d₁, at one end and a greater distance d₂ at the other end, such that the flange 12 is not parallel to the bone engaging surface 6.

Turning now to Figure 3, the flange 12 may be of circular cross section, but is preferably of elongate cross-section, such that its width w₁ is greater than its height h. Most preferably the cross-section of the flange 12 is defined by a segment of a circle c₁ adjoining and coincident with a segment of a circle c₂ of greater diameter.

In order to implant the prosthesis, the proximal end of the tibia is partially cut away to form a flat surface which can be disposed substantially perpendicular to the longitudinal axis of the tibia or sloping posteriorly at an angle of 2 to 7°. A hole, of a diameter substantially equal to the width w₁ of the flange 12, is then drilled antero posteriorly into the proximal end of the tibia, starting at a preset distance d₃ below the anterior edge of the flat surface formed on the tibia and running at an angle ∝ of 2 to 4° away from this flat surface. Preferably, the angle ∝ is chosen such that it is equal to the angle of offset of the flange 12 away from the bone engaging surface 6, and the distance d₃ is preferably chosen such that it is slightly less than the distance d₂ referred to above. A cut of a width substantially equal to the width w₂ of the web 14 is then made from the flat surface of the tibia into the hole.

The end of the flange 12 which is the greater distance d₂ from the bone engaging surface 6 of the body portion 2 is inserted into the hole and the prosthesis is driven across the flat surface formed on the tibia until the entire flange 12 is anchored in the tibia. Prior to insertion of the flange 12 into the tibia, bone cement may be applied to the bone engaging region 4.

It will be appreciated that, as the prosthesis is driven across the tibia, the bone engaging surface 6 is gradually pulled into contact with the flat surface on the tibia because of the offsetting of the hole in the tibia. Thus, the implanted prosthesis is firmly embedded in the tibia. As the distance d₃ is slightly less than the distance d₂ the prosthesis will bottom out on the tibia slightly before it has been driven fully into position. Consequently, the final positioning of the prosthesis has the effect of prestressing it into firm contact with the tibia, so that even where bone cement is used, the quantity of cement need only be minimal.

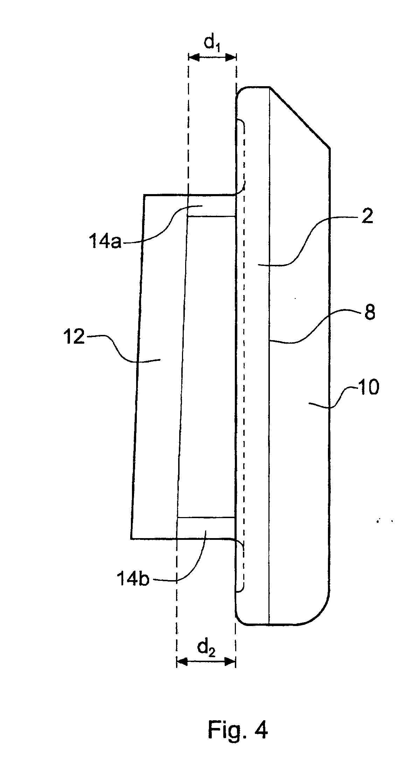

In a further embodiment shown in Figure 4, the web 14 is discontinuous, and comprises a pair of partial webs 14a, 14b of unequal length. Subsequent bone growth in the region defined between the partial webs improves the anchorage of the prosthesis over time.

In a further embodiment, the bone engaging region 4 extends over the same area as the bone engaging surface 6. In other words, the prosthesis lacks the raised border which can be used to retain bone cement between the prosthesis and the tibia and which defines the bone engaging surface 6 in the embodiment of the invention illustrated in Figures 1 to 3.

It is to be understood that the above detailed description of an embodiment of the invention is provided by way of example only. Various details of design and construction may be modified without departing from the true scope of the invention as set forth in the appended claims.

## Claims

1. A prosthesis for attachment to a bone, the prosthesis comprising:
a body portion having a substantially flat bone engaging region;
a flange;
and a web which connects the flange to the bone engaging region of the body portion and spaces it away from the bone engaging region in such a way that the flange is not parallel to the bone engaging region.

2. A prosthesis as claimed in claim 1, in which the flange is substantially straight.

3. A prosthesis as claimed in claim 1 or 2, in which the bone engaging region comprises a plurality of bone engaging surfaces disposed substantially in a common plane.

4. A prosthesis as claimed in any one of claims 1 to 3, in which the widest part of the web is narrower than the widest part of the flange when viewed in a plane perpendicular to a longitudinal axis of the flange.

5. A prosthesis as claimed in any one of the preceding claims, in which the web is tapered in a direction parallel to a longitudinal axis of the flange, so that the flange is spaced further from the bone engaging surface at one end than at the other.

6. A prosthesis as claimed in any one of the preceding claims, in which the flange is of circular cross section.

7. A prosthesis as claimed in any one of the preceding claims, in which the flange has a cross-section defined by a segment of a circle adjoining and coincident with a segment of a circle of greater diameter.

8. A prosthesis as claimed in any one of the preceding claims, in which the web is continuous.

9. A prosthesis as claimed in any one of the preceding claims, in which the bone engaging region is coated in a porous coating and/or hydroxyapatite material.

10. A prosthesis as claimed in any one of the preceding claims, in which the prosthesis is intended for attachment to the superior end of a tibia.
